(19) Europäisches Patentamt
European Patent Office
Office européen des brevets

(11) Veröffentlichungsnummer: **0 065 708**
**A2**

(12) # EUROPÄISCHE PATENTANMELDUNG

(21) Anmeldenummer: 82104157.1

(22) Anmeldetag: 12.05.82

(51) Int. Cl.$^3$: C 07 C 45/60
C 07 C 49/403
//C07D309/32

(30) Priorität: 21.05.81 CH 3321/81

(43) Veröffentlichungstag der Anmeldung:
01.12.82 Patentblatt 82/48

(84) Benannte Vertragsstaaten:
AT BE CH DE FR GB IT LI LU NL SE

(71) Anmelder: LONZA AG

Gampel/Wallis(CH)

(72) Erfinder: Lehky, Pavel, Dr.
Dammweg 13
Naters (Kanton Wallis)(CH)

(72) Erfinder: Kronig, Peter, Dr.
Napoleonstrasse 26
Visp (Kanton Wallis)(CH)

(74) Vertreter: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Verfahren zur Herstellung von Dimedon.**

(57) Dimedon wird aus der 3,3-Dimethyl-5-oxo-hexansäure, welche ihrerseits aus Isophoron über dessen Ozonanlagerungsprodukt und nachfolgender Hydrolyse hergestellt werden kann, durch deren Dehydratisierung zum entsprechenden 4,4,6-Trimethyl-3,4-dihydro-1,2-pyron und durch Behandlung des letzteren mit einem Alkalialkoholat erhalten.

EP 0 065 708 A2

## Verfahren zur Herstellung von Dimedon

Die Erfindung betrifft ein Verfahren zur Herstellung von Dimedon aus 3,3-Dimethyl-5-oxo-hexansäure über deren Lacton, das 4,4,6-Trimethyl-3,4-dihydro-1,2-pyron, als Zwischenprodukt:

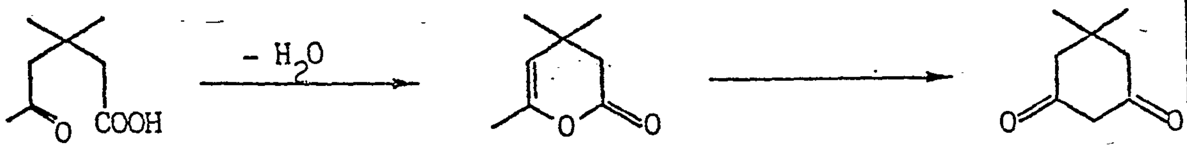


3,3-Dimethyl-5-oxo-hexansäure

4,4,6-Trimethyl-3,4-dihydro-1,2-pyron

Dimedon
5,5-Dimethyl-1,3-cyclohexandion

Dimedon findet als Zwischenprodukt in der chemischen Industrie, wie bei der Herstellung von Pharmazeutika (US-PS 3 775 435, US-PS 3 823 164), Herbiziden resp. Pestiziden (DE-OS 22 01 668, US-PS 3 976 785) und Polymerisaten (JP-PS 77 47949), in der Photochemie (US-PS 2 944 899, JP-PS 78 33143) und in der Analytik (DE-OS 25 12 586) vielseitige Anwendungsmöglichkeiten.

Für die Herstellung von Dimedon sind aus der Literatur verschiedene Methoden bekannt. So wird in Organic Syntheses, Coll. II, 200 (1943) ein Verfahren erwähnt, welches von Malonester und Mesityloxid ausgeht.**) G.B.Payne, J.Org.Chem. 24, 719 (1959) stellt Dimedon aus Isophoron mit Hilfe eines mehrstufigen, komplizierten Verfahrens unter Verwendung von Wasserstoffsuperoxid her. Weiter beschreiben M.Qudrat-I-Khuda, J.Chem.Soc. 1929, 201, und R.Semet et al, Bull.Chim.Soc.France 1978 (3-4), II 185, eine Reaktion in der von der 3,3-Dimethyl-5-oxo-hexan-

**) T.Henshall et al., J.Amer.Chem.Soc. 77, 6656 (1955) erhalten Dimedon durch Zyklisation von 3,3-Dimethyl-5-oxohexansäure mit Schwefelsäure.

säure, unter Bildung eines Lactons, mit Hilfe einer stöchiometrischen Menge oder mit überschüssigem Essigsäureanhydrid Wasser abgespalten wird.

Alle diese Verfahren haben verschiedene Nachteile, wie tiefe Ausbeuten oder viel anorganischen Abfall, oder die Verwendung des rel. teuren Wasserstoffsuperoxides oder, im zuletzt genannten Fall, die Anwendung einer zumindest stöchiometrischen Menge von Essigsäureanhydrid.

Ziel der vorliegenden Erfindung ist es, ausgehend von einem billigen Edukt Dimedon von hoher Reinheit und in einer grossen Ausbeute zu produzieren, ohne dass noch aufwendige Reinigungsverfahren angewendet werden müssen.

Erfindungsgemäss wird dies dadurch erreicht, dass man von 3,3-Dimethyl-5-oxo-hexansäure ausgeht, welche u.a. aus Isophoron über ein Ozonanlagerungsprodukt und nachfolgender Hydrolyse des letzteren erhalten werden kann, und diese 3,3-Dimethyl-5-oxo-hexansäure in einer ersten Stufe durch Wasserabspaltung in das entsprechende 4,4,6-Trimethyl-3,4-dihydro-1,2-pyron überführt und aus letzterem durch Behandlung mit einem Alkalialkoholat das Dimedon bildet.

Die Dehydratisierung der 3,3-Dimethyl-5-oxo-hexansäure kann sowohl durch azeotrope Destillation in Anwesenheit eines Lösungsmittels, zweckmässig von Toluol oder Benzol, vorzugsweise Toluol, mit Hilfe einer geringen Menge einer starken, insbesonde konzentrierten Säure, /vorzugsweise Schwefelsäure, als auch durch Anwendung von Vakuum

in Anwesenheit katalytischer Mengen einer starken, gegebenenfalls konzentrierten Säure, vorzugsweise Schwefelsäure, durchgeführt werden.

Als starke Säure können auch stark saure Ionenaustauscher zur Anwendung kommen.

Als Lösungsmittel kommen alle diejenigen in Frage, die mit Wasser ein Azeotop bilden und im Bereich von 60 bis 150°C sieden.

Wird für die Dehydratisierung der Vakuumweg gewählt, so wird insbesondere bei 5 bis 300 mbar, vorzugsweise bei 10 bis 20 mbar, gearbeitet.

Die Temperaturen, bei welchen der Vakuumweg durchgeführt wird, betragen insbesondere 100 bis 170°C, vorzugsweise 130 bis 150°C, während diejenige bei der azeotropen Destillation durch den Siedepunkt des azeotropen Gemisches Wasser/Lösungsmittel bestimmt wird.

Als starke Säuren kommen in Frage: organische oder anorganische Protonen, die im Wasser ganz oder zumindest weitgehend dissoziert sind ($P_K < 2$) und die bei Reaktionstemperaturen keine oder nur eine geringe Flüchtigkeit aufweisen, wie Schwefelsäure, Phosphorsäure, organische Sulfonsäure, wie p-Toluolsulfonsäure, oder Trichloressigsäure.

Die Dauer der Dehydration, d.h. bis kein Reaktionswasser mehr abgeschieden wird, ist im Falle der azeotropen Destillation vom gewählten Lösungsmittel abhängig und beträgt, im Falle dass Toluol gewählt wird, ca. 16 Stunden und im Falle des Vakuumweges 2 bis 15 Stunden, je nach Reaktionstemperatur.

Die Ueberführung des durch die Dehydration der 3,3-Dimethyl-5-oxo-hexansäure erhaltenen 4,4,6-Trimethyl-3,4-dihydro-1,2-pyrons in Dimedon wird nach einer Ausführungsform der Erfindung mit einem Alkalialkoholat, vorzugsweise mit Natriummethylat, gelöst in Methanol, bei Rückflusstemperaturen durchgeführt, und zwar so, dass das 4,4,6-Trimethyl-3,4-dihydro-1,2-pyron langsam in die vorbereitete, im Rückfluss kochende Alkoholatlösung zugegeben wird. Die Reaktionsdauer beträgt ca. 0,1 bis 5 Stunden, im Mittel ca. 2 bis 3 Stunden, wobei jedoch auch längere Zeiten unschädlich sind.

Nach dieser Reaktionszeit wird die Reaktionslösung auf Raumtemperatur abgekühlt und der pH-Wert zweckmässig mit wässriger Salzsäure auf ca. 1 eingestellt. Dabei fällt ein farbloser Niederschlag aus. Der Alkohol wird abdestilliert und die farblosen Kristalle des Dimedons werden abfiltriert und bei 40°C im Vakuum getrocknet.

Vorzugsweise wird das Ausgangsmaterial, die 3,3-Dimethyl-5-oxo-hexansäure, aus Isophoron hergestellt. Dabei wird Isophoron beispielsweise durch Behandlung mit Ozon in ein Ozonanlagerungsprodukt übergeführt und aus letzterem durch Hydrolyse die Säure gebildet.

Beispiel 1

Dehydration der 3,3-Dimethyl-5-oxo-hexansäure durch azeotrope Destillation.

In einem Rundkolben, welcher mit einem Wasserabscheider versehen war, wurde, 8,047 g 3,3-Dimethyl-5-oxo-hexansäure in

150 ml Toluol vorgelegt und mit 5 Tropfen konz. Schwefelsäure versetzt. Diese Mischung wurde anschliessend so lange auf Siedehitze gehalten, bis kein Wasser mehr abgeschieden wurde. Dies dauerte ca. 16 Stunden. Dann wurde die Reaktionslösung mit 50 ml Wasser gewaschen und in einem Rotationsverdampfer vom Toluol befreit.

Es wurden 6,581 g einer farblosen Flüssigkeit erhalten, die laut spektroskopischer Analyse aus 4,4,6-Trimethyl-3,4-dihydro-1,2-pyron bestand; Gehalt laut gaschromatographischer Analyse 97,62%.

Dies entsprach 6,424 g 100%iger Substanz, was wiederum einer Ausbeute von 90,0%, berechnet auf die eingesetzte 3,3-Dimethyl-5-oxo-hexansäure, gleichkommt.

<u>Beispiel 2</u>

Dehydration der 3,3-Dimethyl-5-oxo-hexansäure mittels Säurekatalyse.

In einer Destillationsapparatur, welche mit einer Vigreux-Kolonne ausgerüstet worden ist, wurden 104,31 g 3,3-Dimethyl-5-oxo-hexansäure und 3 g Schwefelsäure vorgelegt und unter einem Vakuum von 18 mbar erhitzt. Dabei wurde bei einer Badtemperatur von 130 bis 150°C das Lacton, das 4,4,6-Trimethyl-3,4-dihydro-1,2-pyron, gebildet, welches bei 70°C bei einem Vakuum von 18 mbar überdestillierte.

Das angestrebte Lacton, welches mit dem Reaktionswasser zwei Phasen bildete, wurde nach Abtrennung der Wasserphase in einer Menge von 85,45 g erhalten und wies eine Reinheit von 98,7%

auf. Dies entsprach 84,34 g 100%igem 4,4,6-Trimethyl-3,4-dihydro-1,2-pyron, was einer Ausbeute von 91,3%, bezogen auf die eingesetzte 3,3-Dimethyl-5-oxo-hexansäure, gleichkommt.

Beispiel 3

In einem Rundkolben, ausgerüstet mit einem Rückflusskühler und einem Tropftrichter, wurde eine vorbereitete Natriummethylat-Lösung, bestehend aus 1,8 g Natrium und 30 ml Methanol, vorgelegt und auf Rückflusstemperatur erhitzt. Unter Kochen der genannten Lösung am Rückfluss wurden während 30 Minuten 9,331 g Lacton dazugetropft. Nach weiterer 2-stündiger Behandlung bei Rückflusstemperatur wurde die Reaktionslösung auf Raumtemperatur abgekühlt und der pH-Wert mit wässriger Salzsäure (8 ml konz. HCl in 100 ml Wasser) auf ca. 1 eingestellt. Dabei fiel das Dimedon als farbloser Niederschlag aus. Anschliessend wurde bei Normaldruck der Methylalkohol abdestilliert und die nach dem neuerlichen Abkühlen wieder ausfallenden farblosen Dimedon-Kristalle abfiltriert und im Vakuumtrockenschrank bei 40°C getrocknet. Man erhielt 7,634 g Dimedon, Smp. 146,6 bis 148,2°C, Gehalt 98,2% (laut potentiometrischer Titration), entsprechend 7,497 g 100%ig, entsprechend einer Ausbeute von 80,3%, bezogen auf eingesetztes 4,4,6-Trimethyl-3,4-dihydro-1,2-pyron.

Beispiel 4

In einem 100 ml-Rundkolben mit 30 cm Vigreux-Kolonne und Destillationsaufsatz wurden 24,9 g (157 mmol) 3,3-Dimethyl-5-oxo-hexansäure und 2,5 g eines stark sauren Ionenaustauschers (Naphion H) vorgelegt und unter einem Vakuum von 20 mbar erhitzt. Bei einer Badtemperatur von 150°C bildete sich das 4,4,6-Trimethyl-3,4-dihydro-1,2-pyron, welches bei 70 bis 80°C und 20 mbar überdestillierte.

Das angestrebte Produkt, welches mit dem Reaktionswasser zweiphasig vorlag, wurde nach Abtrennung der Wasserphase in einer Menge von 18,8 g erhalten und wies eine Reinheit (GC) von 98,4% auf. Dies entsprach 18,5 g 100%igem 4,4,6-Trimethyl-3,4-dihydro-1,2-pyron, was einer Ausbeute von 84% bezüglich eingesetzter 3,3-Dimethyl-5-oxo-hexansäure gleichkommt.

## Patentansprüche

1. Verfahren zur Herstellung von Dimedon aus 3,3-Dimethyl-5-oxo-hexansäure, dadurch gekennzeichnet, dass man 3,3-Dimethyl-5-oxo-hexansäure in einer ersten Stufe durch Wasserabspaltung in das 4,4,6-Trimethyl-3,4-dihydro-1,2-pyron überführt und letzteres in einer weiteren Stufe durch Behandlung mit einem Alkalialkoholat in das Dimedon überführt.

2. Verfahren gemäss Patentanspruch 1, dadurch gekennzeichnet, dass die Dehydratisierung der 3,3-Dimethyl-5-oxo-hexansäure mit Hilfe einer azeotropen Destillation in Gegenwart geringer Mengen einer starken Säure und in Anwesenheit eines Lösungsmittels, das mit Wasser ein Azeotrop bildet, durchgeführt wird.

3. Verfahren gemäss Patentanspruch 1, dadurch gekennzeichnet, dass die Dehydratisierung der 3,3-Dimethyl-5-oxo-hexansäure in Gegenwart einer starken Säure mit Hilfe von Vakuum, zweckmässig in einem Vakuum von 5 bis 300 mbar, vorzugsweise bei 10 bis 20 mbar, durchgeführt wird.

4. Verfahren gemäss Patentansprüchen 1 bis 3, dadurch gekennzeichnet, dass man die Dehydratisierung der 3,3-Dimethyl-5-oxo-hexansäure in Gegenwart von konzentrierter Schwefelsäure durchführt.

5. Verfahren gemäss Patentanspruch 3, dadurch gekennzeichnet, dass man die Dehydratisierung zweckmässig bei Temperaturen von 100 bis 170°C, vorzugsweise bei 130 bis 150°C, ausführt.

6. Verfahren gemäss Patentansprüchen 1-5, dadurch gekennzeichnet, dass man als das zur Ueberführung des 4,4,6-Trimethyl-3,4-dihydro-1,2-pyrons in Dimedon benötigte Alkalialkoholat Natriummethanolat verwendet.

7. Verfahren gemäss Patentansprüchen 1-6, dadurch gekennzeichnet, dass zwecks Umsetzung des 4,4,6-Trimethyl-3,4-dihydro-1,2-pyrons in Dimedon das 4,4,6-Trimethyl-3,4-dihydro-1,2-pyron zu einer vorbereiteten methanolischen Natriummethanolat-Lösung zugegeben wird.

8. Verfahren nach Patentansprüchen 1 bis 7, dadurch gekennzeichnet, dass die 3,3-Dimethyl-5-oxo-hexansäure aus Isophoron über dessen Ozonanlagerungsprodukt und durch nachfolgende Hydrolyse des letzteren hergestellt wird.